# EUROPEAN PATENT APPLICATION

(11) **EP 4 834 784 A1**
(43) Date of publication of application: **04.11.2026**
(21) Application number: 26172138.5
(22) Date of filing: 14.04.2026
(51) Int. Cl.: B65B 55/10, A61L 2/206, B65B 55/02, A61L 2/208, B65B 9/20, B65B 65/00

(54) **STERILE GAS CONDITIONING APPARATUS, PACKAGING MACHINE FOR FORMING PACKAGES FILLED WITH A POURABLE PRODUCT HAVING A STERILE GAS CONDITIONING APPARATUS AND METHOD FOR CONDITIONING A STERILE GAS**

(30) Priority: 28.04.2025 IT 202500009384
(71) Applicant: Tetra Laval Holdings & Finance S.A., 1009 Pully (CH)
(72) Inventor: DONATI, Andrea, 41123 Modena (IT); CARRAROLI, Piero Luigi, 41123 Modena (IT)
(74) Representative: Tetra Pak Patent Attorneys

(57) **Abstract**

There is described a sterile gas conditioning apparatus (20; 20') comprising a flow circuit (21) having an inlet opening (22) configured to receive a gas exiting from the inner environment (5) and an outlet opening (23), a cleaning device (24) configured to remove particles and/or chemical compounds from the gas, an aspiration device (25) configured to generate an aspiration force for directing at least the gas through the inlet opening (22) and into the flow circuit (21) and at least one separation device (26) configured to separate a liquid phase from the gas. The separation device (26) comprises a collection portion (27) for collecting the liquid phase. The sterile gas conditioning apparatus (20; 20') comprises at least one tubing arrangement (30) fluidically connecting the connection portion (27) with an intake opening (31) of the flow circuit (21) creating a closed recycle circuit (32) between a portion of the flow circuit (21) and the collection portion (27). The sterile gas conditioning apparatus (20; 20') also comprises a tank (50) configured to store a sterilizing agent and a connection tube (51) fluidically connecting the tank (50) to the closed recycle circuit (32) so as to allow for an injection of the sterilizing agent at an agent injection station (52) into the closed recycle circuit (32).

## Description

### TECHNICAL FIELD

The present invention relates to a sterile gas conditioning apparatus for a packaging machine for forming packages of a pourable product, preferentially a pourable food product.

The present invention also relates to a packaging machine for forming packages filled with a pourable product, preferentially a pourable food product, having a sterile gas conditioning apparatus.

Advantageously, the present invention also relates to a method for conditioning a sterile gas of a packaging machine, in particular packaging machine for forming packages filled with a pourable product, preferentially a pourable food product.

### BACKGROUND ART

As is known, many liquid or pourable food products, such as fruit juice, UHT (ultra-high-temperature treated) milk, wine, tomato sauce, etc., are sold in packages made of sterilized packaging material.

A typical example is the parallelepiped-shaped package for liquid or pourable food products known as Tetra Brik Aseptic (registered trademark), which is made by sealing and folding laminated strip packaging material. The packaging material has a multilayer structure comprising a base layer, e.g. of paper, covered on both sides with layers of heat-seal plastic material, e.g. polyethylene. In the case of aseptic packages for long-storage products, such as UHT milk, the packaging material also comprises a layer of oxygen-barrier material (an oxygen-barrier layer), e.g. an aluminum foil, which is superimposed on a layer of heat-seal plastic material, and is in turn covered with another layer of heat-seal plastic material forming the inner face of the package eventually contacting the food product.

Packages of this sort are normally produced on fully automatic packaging machines, which advance a web of packaging material through a sterilization apparatus for sterilizing the web of packaging material at a sterilization station and to an isolation chamber (having a sterile inner environment) in which the sterilized web of packaging material is maintained and advanced. During advancement of the web of packaging material through the isolation chamber, the web of packaging material is folded and sealed longitudinally at a tube forming station to form a tube having a longitudinal seam portion, the tube being further fed along a vertical advancing direction.

For completing the forming operations, the tube is filled with a pourable product, in particular a pourable food product, and is transversally sealed and subsequently cut along equally spaced transversal cross sections within a package forming apparatus of the packaging machine during advancement along the vertical advancing direction.

Pillow packages are so obtained, each pillow package having a longitudinal sealing band, a top transversal sealing band and a bottom transversal sealing band.

A typical packaging machine comprises a conveying device for advancing the web of packaging material along a web advancement path and the tube formed from the web of packaging material along a tube advancement path, the sterilization apparatus for sterilizing the web of packaging material prior to its formation into the tube, a tube forming and sealing device at least partially arranged within the isolation chamber and being configured to form the tube from the advancing web of packaging material and to longitudinally seal the tube, a filling device for filling the tube with the pourable product and the package forming apparatus adapted to form, transversally seal and cut individual packages from the tube of packaging material.

A typical packaging machine also comprises a sterile gas conditioning apparatus for providing a sterile gas, in particular sterile air, into the inner environment. The sterile gas conditioning apparatus defines together with the inner environment and possibly with an inner space of the sterilization apparatus a closed circuit. Thereby, a flow of the sterile gas is guaranteed from the inner environment into the inner space then into the sterile gas conditioning apparatus and back into the inner environment.

In particular, sterile gas conditioning apparatus is configured to guarantee the needed sterility of the sterile gas.

In order to guarantee the sterility of the air fed into the inner environment, the sterile gas conditioning apparatus comprises an inlet opening for receiving the air, a cleaning device for removing particles and/or chemical compounds from the air, an aspiration device for generating the aspiration force, a separator for separating a liquid phase from the air and having a draining outlet for the liquid phase and a sterilization device for sterilizing the air. At least in some solutions, the aspiration device receives, in use, water so as to generate a liquid ring seal.

Even though the known packaging machines and/or sterile gas conditioning apparatus work satisfyingly well, a need is felt in the sector to further improve the known packaging machines and/or sterile gas conditioning apparatus.

### DISCLOSURE OF INVENTION

It is therefore an aim of the present invention to provide an improved sterile gas conditioning apparatus for a packaging machine for forming packages filled with a pourable product, preferentially a pourable food product.

Advantageously, it is a further aim of the present invention to provide an improved packaging machine for forming packages filled with a pourable product, preferentially a pourable food product, having a sterile gas conditioning apparatus.

According to the present invention, there are provided a sterile gas conditioning apparatus and a method as claimed in the respective independent claims.

Preferred non-limiting embodiments of are claimed in the respective dependent claims.

Additionally, according to the present invention, there is provided a packaging machine according to claim 12 or 13.

### BRIEF DESCRIPTION OF THE DRAWINGS

Two non-limiting embodiments of the present invention will be described by way of example with reference to the accompanying drawings, in which:
Figure 1 is a schematic view of a packaging machine having a sterile gas conditioning apparatus according to a first embodiment of the present invention, with parts removed for clarity;
Figure 2 is a schematic view of a detail of the sterile gas conditioning apparatus of Figure 1, with parts removed for clarity; and
Figure 3 is a schematic view of a detail of a sterile gas conditioning apparatus according to a second embodiment of the present invention, with parts removed for clarity.

### BEST MODES FOR CARRYING OUT THE INVENTION

The elements and features illustrated in the preferred embodiment, including drawings, may be combined with each other without going beyond the scope of protection of this application as described below.

Number 1 indicates as a whole a packaging machine for producing packages, preferentially sealed packages, of a pourable food product, in particular a pourable food product, such as milk, milk drinks, yoghurt, yoghurt drinks, fruit juice, wine, tomato sauce, emulsions, beverages containing pulp, salt, sugar, etc.

Packaging machine 1 may be configured to form packages from a tube 2 obtained from a web 3 of packaging material. Preferentially, in use, tube 2 extends along a longitudinal axis, more preferentially having a vertical orientation.

The packaging material forming web 3 may have a multilayer structure, preferentially having heat-seal properties (i.e. portions of the multilayer packaging material can be sealed to one another).

In further detail, the multilayer packaging material may comprise at least one layer of fibrous material, such as e.g. paper or cardboard, and at least two layers of heat-seal plastic material, e.g. polyethylene, interposing the layer of fibrous material in between one another. Preferentially, one of these two layers of heat-seal plastic material may define the inner face of the packages contacting the pourable product.

Moreover, the multilayer packaging material may also comprise a layer of gas- and light-barrier material, e.g. aluminum foil or ethylene vinyl alcohol (EVOH) film, preferentially being arranged between one of the layers of heat-seal plastic material and the layer of fibrous material.

Preferentially, the packaging material may also comprise a further layer of heat-seal plastic material being interposed between the layer of gas- and light-barrier material and the layer of fibrous material.

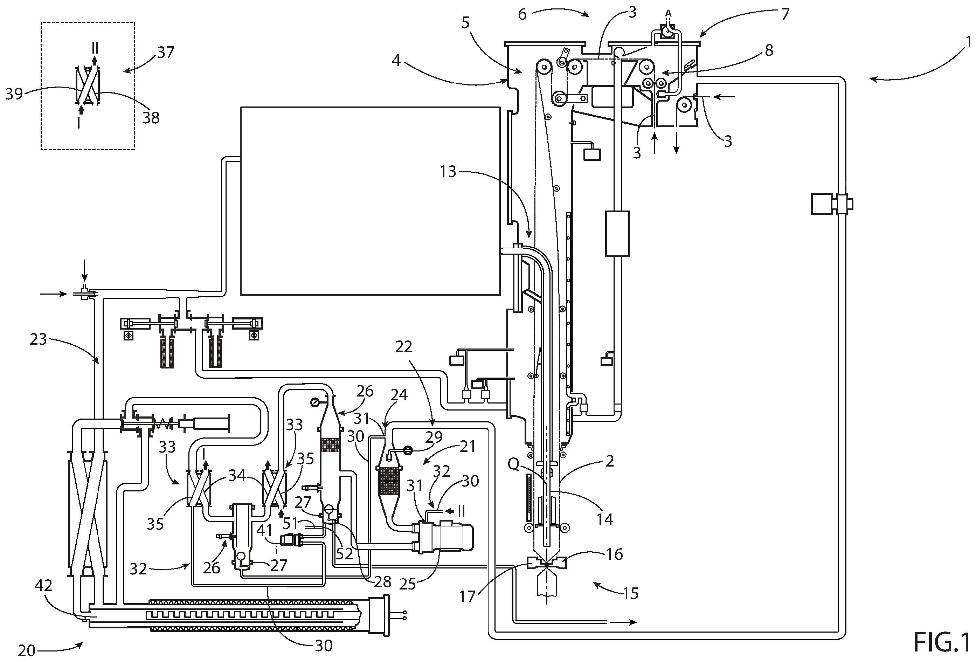

With particular reference to Figure 1, packaging machine 1 may comprise an isolation chamber 4 having an inner environment 5, e.g. containing a sterile gas, more specifically sterile air.

In more detail, isolation chamber 4 divides inner environment 5 from an outer environment 6.

In particular, isolation chamber 4 allows to package the pourable product under sterile (aseptic) conditions.

Furthermore, packaging machine 1 may comprise a sterilization apparatus 7 (only partially shown) for sterilizing the packaging material, more specifically web 3. More specifically, sterilization apparatus 7 may be configured to sterilize the packaging material, more specifically web 3.

In more detail, sterilization apparatus 7 may comprise an inner space 8 within which, in use, the packaging material, more specifically web 3, is sterilized.

According to some possible embodiments, sterilization apparatus 7 may comprise a sterilization unit arranged within inner space 8 configured to sterilize the packaging material.

For example, the sterilization unit may be configured to sterilize the packaging material by chemical sterilization such as by means of hydrogen peroxide.

Alternatively or in addition, the sterilization unit may be configured to sterilize the packaging material by means of physical sterilization such as electron beam sterilization.

Additionally, inner space 8 and inner environment 5 may be in fluid connection with one another.

Moreover, in use, the packaging material, more specifically web 3, may enter inner environment 5 through and from inner space 8 (in other words, the packaging material, more specifically web 3, is at first sterilized and then introduced into inner environment 5).

In further detail, packaging machine 1 may comprise a conveying device configured to advance web 3 along a web advancement path, more specifically from a delivery station to a tube forming station, at which, in use, web 3 is formed into tube 2. The conveying device may also be configured to advance tube 2 along a tube advancement path Q.

Packaging machine 1 may comprise a tube forming and sealing device at least partially arranged within inner environment 5 and being configured to form tube 2, more specifically to fold tube 2 from web 3, and to longitudinally seal tube 2. The tube forming and sealing device is preferentially arranged at tube forming station.

In more detail, the tube forming and sealing device may be at least partially arranged within isolation chamber 4, more specifically inner environment 5, and may be configured to form and longitudinally seal tube 2 within isolation chamber 4, more specifically inner environment 5.

In further detail, the tube forming and sealing device may be configured to gradually fold web 3 into tube 2 by overlapping a first lateral edge of web 3 and a second lateral edge of web 3 with one another for forming a longitudinal seam portion of tube 2. Preferentially, the tube forming and sealing device may be configured to longitudinally seal the longitudinal seam portion.

More specifically, the conveying device may be configured to advance tube 2 and any intermediate of tube 2 along tube advancement path Q. Even more specifically, with the wording intermediates of tube 2 any configuration of web 3 is meant prior to obtaining the tube structure and after folding of web 3 by the tube forming and sealing device has started. In other words, the intermediates of tube 2 are a result of the gradual folding of web 3 so as to obtain tube 2, preferentially by overlapping with one another the first lateral edge of web 3 and the second lateral edge of web 3.

Additionally, packaging machine 1 may comprise a filling device 13 configured to fill tube 2 with the pourable product.

With particular reference to Figure 1, filling device 13 may comprise a filling pipe 14 being and/or being configured to be put in fluid connection with a pourable product storage tank, which is adapted to store/provide for the pourable product to be packaged.

More specifically, filling pipe 14 may be configured to direct, in use, the pourable product into tube 2. Preferentially, filling pipe 14 may be configured to be, in use, at least partially placed within tube 2 for feeding the pourable product into tube 2.

Packaging machine 1 may also comprise a package forming apparatus 15 configured to manipulate, more specifically to at least partially shape and/or transversally seal and/or transversally cut, tube 2 for obtaining the packages from tube 2.

In more detail, package forming apparatus 15 may be arranged downstream of isolation chamber 4 along tube advancement path Q.

With reference to Figure 1, package forming apparatus 15 may comprise a plurality of operative assemblies 16 (only one being partially shown) and a plurality of counter-operative assemblies 17 (only one being partially shown) configured to manipulate in collaboration with one another tube 2 for forming the packages, more specifically to at least partially shape and/or transversally seal and/or transversally cut, tube 2 for forming the packages.

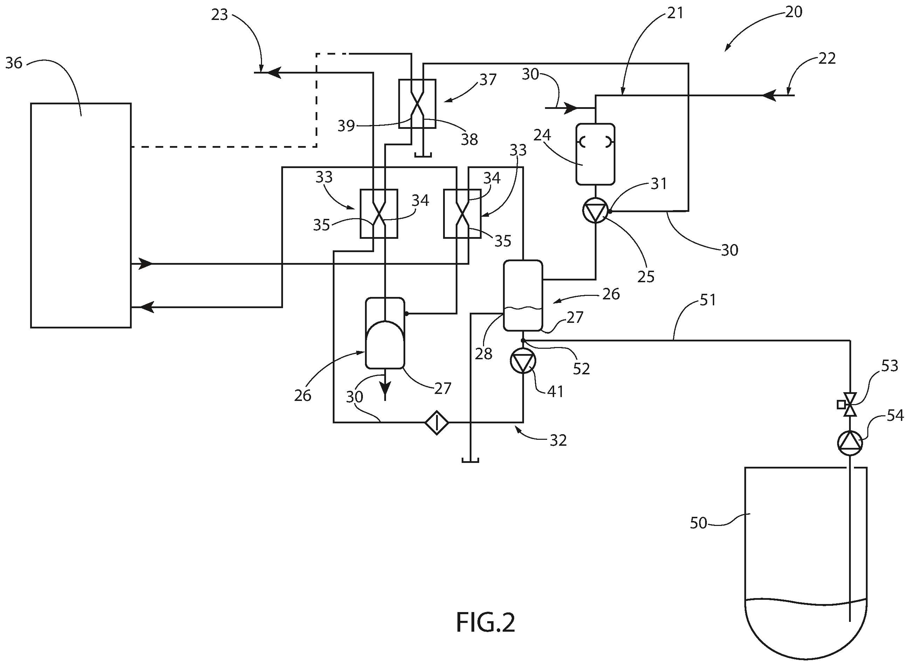

With particular reference to Figures 1 and 2, packaging machine 1 also comprises a sterile gas conditioning apparatus 20 configured to condition a gas, more specifically air, to be used in packaging machine 1, more specifically to be introduced into inner environment 5.

More specifically, sterile gas conditioning apparatus 20 may be configured to treat the gas so as to obtain a sterile gas (which can be introduced into inner environment 5 and which corresponds to defined hygienic standards, i.e. fulfils defined hygienic requirements).

In more detail, sterile gas conditioning apparatus 20 may be configured to produce a sterile gas, more specifically sterile air, to be used within packaging machine 1, more specifically to be introduced into inner environment 5.

According to some non-limiting embodiments, at least portions of sterile gas conditioning apparatus 20 may define a closed gas flow circuit together with portions of packaging machine 1, more specifically together with inner environment 5 and inner space 8.

Advantageously, the gas conditioned by sterile conditioning apparatus 20 may be injected into inner environment 5 at one or more injection stations.

According to some non-limiting embodiments, packaging machine 1 may comprise a control unit configured to control that a first pressure within inner environment 5 may be greater than ambient pressure and greater than a second pressure within inner space 8. Additionally, the second pressure may be greater than ambient pressure too.

With reference to Figures 1 and 2, sterile gas conditioning apparatus 20 comprises:
- a flow circuit 21 having an inlet opening 22 configured to be in fluidic connection with inner environment 5 and to receive gas, more specifically air, exiting from inner environment 5 and an outlet opening 23 being in fluidic connection and/or being controllable in fluidic connection with inlet opening 22 and being configured to be in fluidic connection with inner environment 5 and to allow to feed the gas, more specifically the air, back into inner environment 5;
- a cleaning device 24 being configured to remove particles and/or chemical compounds from the gas, more specifically the air;
- an aspiration device 25 configured to generate an aspiration force for directing at least the gas, more specifically the air, through inlet opening 22 and into flow circuit 21; and
- one or more separation devices 26 configured to separate a liquid phase (i.e. water) from the gas, more specifically the air (entering separation device 26).

Furthermore, at least respective portions of cleaning device 24, aspiration device 25 and separation device 26 may be integrated into flow circuit 21.

Moreover, the respective portions of cleaning device 24, aspiration device 25 and separation device 26 may be fluidically interposed between inlet opening 22 and outlet opening 23.

In further detail, cleaning device 24 may be arranged downstream of inlet opening 22, aspiration device 25 may be arranged downstream of cleaning device 24, each separation device 26 may be arranged downstream of aspiration device 25 and outlet opening 23 may be arranged downstream of each separation device 26.

For example, cleaning device 24 may be fluidically interposed between inlet opening 22 and aspiration device 25.

For example, aspiration device 25 may be fluidically interposed between cleaning device 24 and the one or more separation devices 26.

For example, the one or more separation devices 26 may be fluidically interposed between aspiration device 25 and outlet opening 23.

With reference to Figures 1 and 2, each separation device 26 may comprise a collection portion 27 for collecting the liquid phase and an outlet aperture configured to allow for the outflow of at least a portion of the liquid phase being, in use, collected within the respective collection portion 27.

According to some possible non-limiting embodiments, sterile gas conditioning apparatus 20, e.g. flow circuit 21, may comprise an injection unit 29 configured to inject a liquid, e.g. water, into cleaning device 24.

In such a case, separation device 26 may allow to at least partially reduce the liquid phase of the gas resulting from the operation of injection unit 29.

Advantageously, sterile gas conditioning apparatus 20 also comprises a tank 50 configured to store a sterilizing agent, e.g. a water-sterilizing agent solution. E.g. the water-sterilizing agent solution may have a concentration of the sterilizing agent of at least 20volume%, preferentially of at least 25volume%, even more preferentially of at least 30volume%, and in particular below 50volume%, even more particular below 40volume%. According to one specific example, the water-sterilizing agent solution may have a concentration of the sterilizing agent of 35volume%.

The sterilizing agent may e.g. be hydrogen peroxide.

Advantageously, sterile gas conditioning apparatus 20 further comprises a connection tube 51 fluidically connecting tank 50 with closed recycle circuit 32 so as to allow for an injection of the sterilizing agent, in particular in liquid form, into closed recycle circuit 32 at an agent injection station 52. In particular, injection of the sterilizing agent occurs, in use, by injecting the water-sterilizing agent solution.

Preferentially, tank 50 may also be used to provide for the sterilizing agent used to sterilize the packaging material and/or used during a sterilization-in-place action.

With particular reference to Figure 2, agent injection station 52 is arranged fluidically downstream from one respective separation device 26. In particular, agent injection station 52 may be interposed between two separation devices 26.

Advantageously, sterile gas conditioning apparatus 20 further comprises a control device configured to selectively activate and disactivate an injection of the sterilization agent at agent injection station 52 into closed recycle circuit 32. In particular, the control device is configured to activate the injection of the sterilization agent at the beginning of operation of packaging machine 1, and in particular such to fill up the closed recycle circuit 32, and even more particular to guarantee a constant flow of liquid to aspiration device 25.

In further detail, sterile gas conditioning apparatus 20 comprises a valve 53 arranged within connection tube 51 and being operatively connected to the control device, with control device being configured to selectively control valve 53 so as to activate or disactivate injection of the sterilization agent into closed recycle circuit 32 at agent injection station 52.

Additionally or alternatively, sterile gas conditioning apparatus 20 comprises a further pump 54 arranged within connection tube 51 and being operatively connected to the control device, the control device being configured to selectively control further pump 54 so as to selectively control injection of the sterilization agent.

Preferably, sterile gas conditioning apparatus 20 comprises a three-way valve (not illustrated in the drawings) between connection tube 51 and auxiliary tube 52. In particular, this three-way valve is controlled by the control unit as a function of a peroxide concentration in tank 50 (the peroxide concentration being detected by a sensor connected to the control unit). The control unit is in fact connected to the peroxide concentration sensor, and is configured to control the three-way valve as a function of the peroxide concentration detected in tank 50. If peroxide concentration in tank 50 exceeds a predetermined threshold value, the three-way valve is controlled to send peroxide with low concentration through connection tube 51 into the tank 50. In this configuration, preferably, no peroxide is sent to auxiliary tube 52. If peroxide concentration in the tank is below a predetermined threshold value, the three-way valve is controlled to send peroxide with low concentration to the auxiliary tube 52, to be drained-out. In this configuration, preferably, no peroxide is sent to the tank 50 through connection tube 51.

Reverting again to Figures 1 and 2, sterile gas conditioning apparatus 20 also comprises one or more tubing arrangements 30, each tubing arrangement 30 fluidically connecting one respective outlet aperture of each separation device 26 with a respective intake opening 31 of the flow circuit 21 creating a respective closed recycle circuit 32 between at least a portion of flow circuit 21 and the respective collecting portion 27.

In this way, it is possible to recycle the liquid phase within flow circuit 21.

More specifically, each intake opening 31 is configured to allow for the introduction of the liquid phases into flow circuit 21.

In the specific embodiment shown, sterile gas conditioning apparatus 20 may comprise two separation devices 26, in the specific case giving rise to two closed recycle circuits 32.

More specifically, separation devices 26 may be arranged fluidically in series (with respect to the flow of the gas). In other words, separation devices 26 may be arranged one after the other along flow circuit 21, i.e. one separation device 26 is arranged upstream of the other separation device 26 along flow circuit 21.

According to some possible non-limiting embodiments, one intake opening 31 may be fluidically interposed between cleaning device 24 and aspiration device 25 (i.e. the liquid phase may enter the flow circuit 21 at a position being interposed between cleaning device 24 and aspiration device 25) and/or intake opening 31 may be associated to aspiration device 25.

According to such an embodiment, the liquid phase is transferred from one respective collecting portion 27 to aspiration device 25.

In more detail, aspiration device 25 may be configured to use the liquid phase for generating, in use, a liquid ring seal.

In even more detail, aspiration device 25 may consist of and/or may comprise a liquid ring compressor and at least a portion of the liquid phase (the water collected in collection portion 27) is used to generate a liquid ring seal of the liquid ring compressor. In other words, at least a portion of the liquid phase (the water collected in collection portion 27) is returned to aspiration device 25 for obtaining the desired liquid ring seal.

According to some possible non-limiting embodiments, one intake opening 31 may be fluidically interposed between inlet opening 22 and cleaning device 24 and/or intake opening 31 may be associated to cleaning device 24.

According to such an embodiment, the liquid phase is transferred from one respective collecting portion 27 to cleaning device 24.

According to some embodiments, flow circuit 21 may comprise more than one intake opening 31. For example, in the specific case shown, at least a portion of the liquid phase collected in one respective collection portion 27 is transferred to cleaning device 24 and at least a portion of the liquid phase collected in another respective collection portion 27 is transferred to aspiration device 25. In particular, at least a portion of the liquid phase collected in the collection portion 27 of the separation device 26 that is arranged upstream of the other separation device 26 along flow circuit 21 is transferred to aspiration device 25. Moreover, at least a portion of the liquid phase collected in the collection portion 27 of the separation device 26 that is arranged downstream of the other separation device 26 along flow circuit 21 is transferred to cleaning device 24.

Alternatively, each intake opening 31 may be fluidically connected to more than one collection portion 27.

With reference to Figures 1 and 2, at least one separation device 26 may comprise an auxiliary aperture 28 configured to allow to drain a portion of the liquid phase present within collection portion 27. By having both the outlet aperture and auxiliary aperture 28 it is possible to reuse (through the outlet aperture) the needed quantity of the liquid phase and drain (through auxiliary aperture 28) the portion of the liquid phases that exceeds the needed quantity.

Additionally, each separation device 26 having an auxiliary aperture 28 may be configured to hold a desired level of the liquid phase, and advantageously, may comprise a control valve associated to the respective auxiliary aperture 28 configured to keep the desired level of the liquid phase.

With particular reference to Figures 1 and 2, sterile gas conditioning apparatus 20 may further comprise one or more main heat exchangers 33, each having at least a first pipe 34 for the gas and a second pipe 35 for a fluid, more specifically for a liquid.

In more detail, first pipes 34 may be part of flow circuit 21 and may be fluidically interposed between inlet opening 22 and outlet opening 23.

Moreover, in use, a heat exchange between the gas flowing within the respective first pipe 34 and the fluid flowing within the respective second pipe 35 occurs.

Additionally, main heat exchangers 33 may be arranged fluidically in series.

In further detail, each main heat exchanger 33 may be arranged fluidically downstream of at least one respective separation device 26. For example, one main heat exchanger 33 may be fluidically interposed between two separation devices 26.

In the specific example shown, sterile gas conditioning unit 20 may comprise two main heat exchangers 33.

In the example shown, a first separation device 26 of the two separation devices 26 is arranged fluidically downstream of cleaning device 24 and aspiration device 25, a first main heat exchanger 33 of the two main heat exchangers 33 is arranged fluidically downstream of the first separation device 26, a second separation device 26 of the two separation devices 26 is arranged fluidically downstream of the first main heat exchanger 33, and a second main heat exchanger 33 of the two main heat exchangers 33 is arranged fluidically downstream of the second separation device 26.

According to the specific example shown, one main heat exchanger 33 may be configured to cool the sterile gas and/or another main heat exchanger 33 may be configured to heat the sterile gas.

More specifically, the main heat exchanger 33 being configured to heat the sterile gas may be arranged downstream of the main heat exchanger 33 being configured to cool the sterile gas. In particular, the main heat exchanger 33 being configured to cool the sterile gas may be coupled to the respective closed recycle circuit 32.

According to the embodiment shown, the main heat exchanger 33 being configured to heat the sterile gas may be configured to exchange heat between the sterile gas and the liquid phase flowing within closed recycle circuit 32.

Moreover, the main heat exchanger 33 (the upstream main heat exchanger 33) being arranged upstream of the other main heat exchanger 33 (the downstream main heat exchanger 33) is designed to recover more liquid phase than the downstream main heat exchanger 33, while the liquid phase recovered by the downstream main heat exchanger 33 has a lower temperature than the liquid phase recovered by the upstream main heat exchanger 33.

According to the specific embodiment shown and with reference to Figure 2, packaging machine 1 may comprise a chilling unit 36.

Additionally, chilling unit 36 may be fluidically coupled to one main heat exchanger 33, more specifically to the respective second pipe 35.

Additionally, the other main heat exchanger 33 is not fluidically connected to chilling unit 36 and may be fluidically coupled to a respective closed recycle circuit 32.

More specifically, the respective first pipe 34 of the main heat exchanger 33 being fluidically coupled to the respective closed recycle circuit 32 may be arranged fluidically downstream of the respective first pipe 34 of the main heat exchanger being 33 fluidically coupled to chilling unit 36.

Furthermore, the main heat exchanger 33 being configured to cool the sterile gas may be configured to cool the sterile gas by heat exchange with water originating from chilling unit 36 and such water may flow, in use, through the respective second pipe 35.

According to some non-limiting embodiments, sterile gas conditioning unit 20 may comprise at least one auxiliary heat exchanger 37 having a first pipe 39 for the liquid phase and a second pipe 38 for water of chilling unit 36, in particular the water may be fed back to chilling unit 36 or may be discarded.

According to some preferred non-limiting embodiments, auxiliary heat exchanger 37 may be configured to cool the liquid phase, in particular by heat exchange between the liquid phase and the wastewater of chilling unit 36.

More specifically, first pipe 39 may be integrated into a respective closed recycle circuit 32.

Thus, at least one main heat exchanger 33 and auxiliary heat exchanger 37 may be arranged such that, respectively, second pipe 35 and first pipe 39 are comprised by a respective closed recycle circuit 32.

Moreover, second pipe 38 may be fluidically coupled to chilling unit 36.

For example, first pipe 39 may be arranged fluidically downstream of the respective second pipe 35 which is integrated into closed recycle circuit 32.

According to some non-limiting embodiments, one separation device 26 may be arranged fluidically upstream (with respect to the flow of the gas) of main heat exchangers 33.

According to some non-limiting embodiments, one separation device 26 may be fluidically interposed (with respect to the flow of the gas) between two main heat exchangers 33, more specifically between the respective first pipes 34.

According to some possible embodiments, at least one of closed recycle circuits 32 may comprise a pumping device 41 configured to generate a pumping force for controlling flow of the respective liquid phase through the respective closed recycle circuit 32.

Advantageously, agent injection station 52 is fluidically interposed between the respective separation device 26 and pumping device 41.

With reference to Figure 1, sterile gas conditioning apparatus 20 may further comprise a sterilization device 42 being configured to sterilize the gas prior to exiting through outlet opening 23.

More specifically, sterilization device 42 may be fluidically interposed between outlet opening 23 and the one or more separation devices 26.

In use, packaging machine 1 forms packages filled with the pourable product.

Packaging machine 1 forms the packages from tube 2 being formed from the web of packaging material 3 within inner environment 5.

In more detail, the conveying device advances web of packaging material 3 along the advancement path and within inner environment 5 within which web of packaging material 3 is formed by the tube forming and sealing device. Additionally, tube 2 is filled by filling device 13 with the pourable product.

In further detail, the conveying device also advances tube 3 along tube advancement path Q.

Package forming apparatus 15 forms and transversally seals tube 3 and, preferentially, also transversally cuts tube 3 so as to obtain the packages.

Prior to entering inner environment 5, web of packaging material 3 is sterilized within inner space 8 and by operation of sterilization apparatus 7.

During operation of packaging machine 1 the sterile gas, more specifically the sterile air, is needed and sterile gas conditioning apparatus 20 sterilizes the gas exiting from inner environment 5 and feeds it after its conditioning back into inner environment 5.

In more detail, the gas exiting from inner environment 5, e.g. passing also through inner space 8, enters sterile gas conditioning apparatus 20.

Cleaning device 24 removes particles and/or chemical compounds from the gas, aspiration device 25 generates the aspiration force for directing at least the gas through inlet opening 22 and into flow circuit 21 and each separation device 26 separates the liquid phase from the gas.

Additionally, each liquid phase collected in the respective collection portion 27 is at least partially fed through the respective intake opening 31 into flow circuit 21.

The gas is fed through one or more main heat exchangers 33.

Finally, sterilizing device 42 sterilizes the gas prior to its reintroduction into inner environment 5.

Advantageously, the sterilizing agent originating from tank 50 is injected into closed recycle circuit 32 at agent injection station 52. In particular, the sterilizing agent is injected into closed recycle circuit 32 at agent injection station 52 at the beginning of the operation of packaging machine 1 and such to obtain a desired sterilization agent volume.

In further detail, a method for conditioning the sterile gas is executed and comprises the steps of:
- circulating, during which the gas exiting from inner environment 5 is introduced through inlet opening 22 into flow circuit 21 and during which the gas is fed through outlet opening 23 of flow circuit 21 and back into inner environment 5, in particular after having been conditioned;
- cleaning, during which particles and/or chemical compounds are removed from the gas during their circulation within the flow circuit 21 by cleaning device 24;

- generating, during which aspiration device 25 generates an aspiration force for directing at least the gas through inlet opening 22 and into flow circuit 21;
- separating, during which at least one separation device 26 separates the liquid phase from the gas circulating within flow circuit 21 and the liquid phase is collected within the respective collection portion 27;
- flowing, during which at least a portion of the liquid phase collected within the respective collection portion 27 flows through closed recycle circuit 32 and from collection portion 27 into flow circuit 21; and
- injecting the sterilizing agent from tank 50 into closed recycle circuit 32.

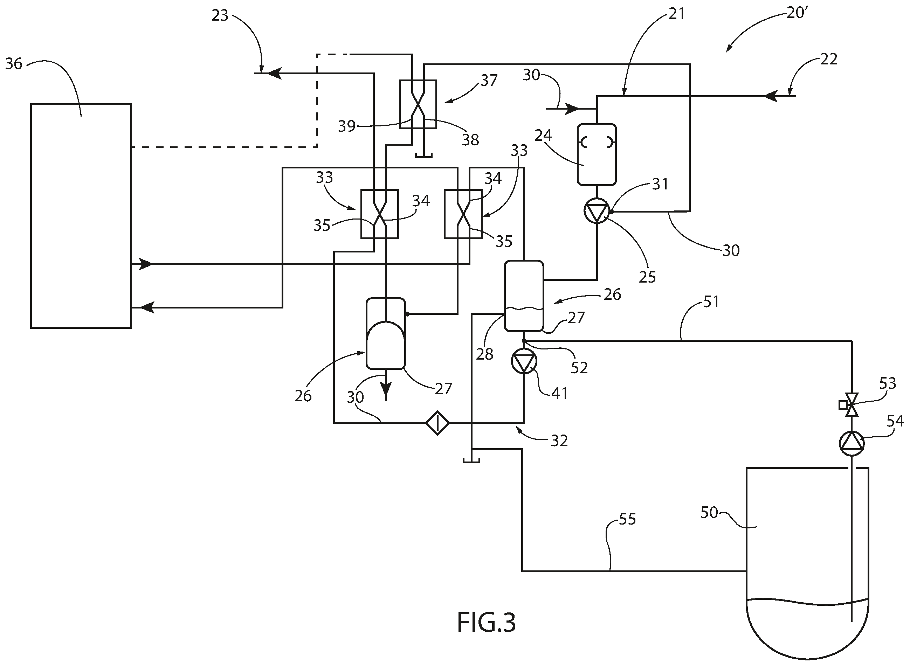

With reference to Figure 3, number 20' indicates an alternative embodiment of a sterile gas conditioning apparatus according to the present invention; as sterile gas conditioning apparatus 20' is similar to sterile gas conditioning apparatus 20, the following description is limited to the differences between them, and using the same references, where possible, for identical or corresponding parts.

In particular, sterile gas conditioning apparatus 20' differs from sterile gas conditioning apparatus 20 in that auxiliary aperture 28 of at least one separation device 26 is configured to allow directing at least a portion of the liquid phase present within the respective collection portion 27 into tank 50.

In particular, sterile gas conditioning apparatus 20' comprises a tube 55 fluidically connecting auxiliary aperture 28 with tank 50.

In the example embodiment of Figure 3, it is shown that a portion of the liquid phase exiting the respective collection portion 27 through auxiliary aperture 28 is directed into tank 50, while another portion is drained. Alternatively, all of the liquid phase exiting the respective collection portion 27 through auxiliary aperture 28 is directed into tank 50.

Accordingly, according to this embodiment, the method of conditioning the sterile gas also comprises the step of feeding, during which at least a portion of the liquid phase present within collection portion 27 is fed, in particular through tube 55, into tank 50.

The advantages of packaging machine 1 according to the present invention will be clear from the foregoing description.

In particular, by collecting the liquid phase in the respective collection portions 27 it is possible to optimize the overall water consumption connected to operation of sterile gas conditioning apparatus 20.

Additionally, the Applicant has observed that by having the possibility of injecting the sterilizing agent into closed recycle circuit 32, it is possible to reduce liming effects.

Furthermore, the Applicant has observed that it is possible to increase the lifetime of components of packaging machine 1. This again means that maintenance activities can be reduced.

Additionally, the use of main heat exchangers 33 and/or auxiliary heat exchanger 37 allows to optimize the energy consumption.

Additionally, a pre-heating of the sterile gas can be obtained thanks to the respective main heat exchanger 33 being configured to heat the sterile gas prior to the sterile gas entering sterilization device 42.

Moreover, it is possible to keep the recycled liquid phase below a threshold temperature prior to feeding the liquid phase back into flow circuit 21 thanks to auxiliary heat exchanger 37.

Additionally, by conveying into the flow circuit 21 the portion of liquid phase recovered from the main heat exchanger 33 arranged downstream of the heat exchanger 33 being configured to cool the sterile gas it is possible to keep the recycled liquid phase below a threshold temperature.

Clearly, changes may be made to packaging machine 1 and/or sterile gas conditioning apparatus 20 as described herein without, however, departing from the scope of protection as defined in the accompanying claims.

According to a possible embodiment, sterile gas conditioning apparatus 20" may not comprise injection device 29.

## Claims

1. Sterile gas conditioning apparatus (20; 20') for a packaging machine (1) for forming packages filled with a pourable product; the sterile gas conditioning apparatus (20; 20') comprises:
- a flow circuit (21) having an inlet opening (22) configured to be in fluidic connection with an inner environment (5) of the packaging machine (1) and to receive a gas exiting from the inner environment (5) and an outlet opening (23) being in fluidic connection and/or being controllable in fluidic connection with the inlet opening (22) and being configured to be in fluidic connection with the inner environment (5) and to allow to feed the gas back into the inner environment (5);
- a cleaning device (24) configured to remove particles and/or chemical compounds from the gas;
- an aspiration device (25) configured to generate an aspiration force for directing at least the gas through the inlet opening (22) and into the flow circuit (21); and
- at least one separation device (26) configured to separate a liquid phase from the gas;
wherein at least respective portions of the cleaning device (24), the aspiration device (25) and the at least one separation device (26) are integrated into the flow circuit (21) and are fluidically interposed between the inlet opening (22) and the outlet opening (23);
wherein the at least one separation device (26) comprises a collection portion (27) for collecting the liquid phase and an outlet aperture configured to allow for the outflow of at least a portion of the liquid phase being, in use, collected within the collection portion (27);
wherein the sterile gas conditioning apparatus (20; 20') comprises at least one tubing arrangement (30) fluidically connecting the outlet aperture with a respective intake opening (31) of the flow circuit (21) creating a closed recycle circuit (32) between a portion of the flow circuit (21) and the collection portion (27);
wherein the sterile gas conditioning apparatus (20; 20') also comprises a tank (50) configured to store a sterilizing agent and a connection tube (51) fluidically connecting the tank (50) to the closed recycle circuit (32) so as to allow for an injection of the sterilizing agent at an agent injection station (52) into the closed recycle circuit (32).

2. Sterile gas conditioning apparatus (20; 20') according to claim 1, wherein the agent injection station (52) is downstream from the at least one separation device (26).

3. Sterile gas conditioning apparatus (20; 20') according to claim 1 or 2, further comprising a pumping device (41) configured to generate a pumping force for controlling flow of the respective liquid phase through the respective closed recycle circuit (32);
wherein the agent injection station (52) is interposed between the at least one separation device (26) and the pumping device (41).

4. Sterile gas conditioning apparatus (20; 20') according to any one of claims 1 to 3, further comprising a control device configured to selectively activate and disactivate an injection of the sterilization agent at the agent injection station (52) into the closed recycle circuit (32).

5. Sterile gas conditioning apparatus (20; 20') according to any one of the preceding claims, wherein the aspiration device (25) is fluidically interposed between the cleaning device (24) and the at least one separation device (26) ;
wherein the intake opening (31) is fluidically interposed between the cleaning device (24) and the aspiration device (25) and/or the intake opening (31) is associated to the aspiration device (25); and/or
wherein the intake opening (31) is fluidically interposed between the inlet opening (22) and the cleaning device (24) and/or the intake opening (31) is associated to the cleaning device (24).

6. Sterile gas conditioning apparatus (20; 20') according to any one of the preceding claims, wherein the at least one separation device (26) comprises an auxiliary aperture (28) configured to allow to drain a portion of the liquid phase present within the collection portion (27) and/or to direct at least a portion of the liquid phase present within the collection portion (27) into the tank (50).

7. Sterile gas conditioning apparatus (20; 20') according to any one of the preceding claims, further comprising and a tube (55) fluidically connecting the tank (50) with the collection portion (27) of the at least one separation device (26);
wherein the tube (55) is configured to allow directing at least a portion of the liquid phase present within the collection portion (27) into the tank (50).

8. Sterile gas conditioning apparatus (20; 20') according to any one of the preceding claims, and further comprising at least one heat exchanger (33) having a first pipe (34) for the gas and a second pipe (35) for a fluid.

9. Sterile gas conditioning apparatus (20; 20') according to any one of the preceding claims, and further comprising a sterilization device (42) fluidically interposed between the outlet opening (23) and the at least one separation device (26) and being configured to sterilize the gas prior to exiting through the outlet opening (23).

10. Sterile gas conditioning apparatus (20; 20') according to any one of the preceding claims, wherein the intake opening (31) is arranged such that the liquid phase is transferred from the respective collection portion (27) to the aspiration device (25) and/or the intake opening (31) is arranged such that the liquid phase is transferred from the respective collection portion (27) to the cleaning device (24) .

11. Sterile gas conditioning apparatus (20; 20') according to any one of the preceding claims, wherein the aspiration device (25) consists of and/or comprises a liquid ring compressor and the liquid phase is used to generate a liquid ring seal of the liquid ring compressor.

12. Packaging machine (1) for forming packages filled with a pourable product:
- an isolation chamber (4) having an inner environment (5); and
- a sterile gas conditioning apparatus (20; 20') according to any one of the preceding claims;
wherein the inlet opening (22) and the outlet opening (23) are fluidically connected to the inner environment (5).

13. Packaging machine (1) according to claim 12 and being configured to form the packages from a packaging material;
wherein the packaging machine (1) comprises a sterilization apparatus (7) configured to sterilize the packaging material within an inner space (8) of the sterilization apparatus (7);
wherein the inner space (8) is arranged such that the packaging material enters into the inner environment (5) from the inner space (8);
wherein the sterile gas conditioning apparatus (20; 20') is configured to receive the gas from the inner environment (5) through the inner space (8) and to direct the gas through the outlet opening (23) into the inner environment (5).

14. Method for conditioning a sterile gas for a packaging machine (1) for forming packages filled with a pourable product;
the method comprises the steps of:
- circulating, during which a gas exiting from an inner environment (5) of the packaging machine (1) is introduced through an inlet opening (22) of a flow circuit (21) into the flow circuit (21) and during which the gas is fed through an outlet opening (23) of the flow circuit (21) and back into the inner environment (5);
- cleaning, during which particles and/or chemical compounds are removed from the gas during their circulation within the flow circuit (21) by a cleaning device (24);
- generating, during which an aspiration device (25) generates an aspiration force for directing at least the gas through the inlet opening (22) and into the flow circuit (21) ;
- separating, during which at least one separation device (26) separates a liquid phase from the gas circulating within the flow circuit (21) and the liquid phase is collected within a collection portion (27) of the separation device (26);
- flowing, during which at least a portion of the liquid phase collected within the collection portion (27) flows through a closed recycle circuit (32) defined between a portion of the flow circuit (21) and the collection portion (27) and from the collection portion (27) into the flow circuit (21); and
- injecting a sterilizing agent from a tank (50) into the closed recycle circuit (32).

15. Method according to claim 14, further comprising the step of feeding, during which at least a portion of the liquid phase present within the collection portion (27) is fed into the tank (50).
